# NEW EUROPEAN PATENT SPECIFICATION

(11) **EP 1 032 336 B2**
(45) Date of publication and mention of the opposition decision: **14.04.2010**
(45) Mention of the grant of the patent: 20.08.2003
(21) Application number: 97949439.0
(22) Date of filing: 14.11.1997
(51) Int. Cl.: A61F 13/15

(54) **DISPOSABLE ABSORBENT ARTICLE WITH A SKIN CARE COMPOSITION ON AN APERTURED TOPSHEET**
ABSORBIERENDER WEGWERFARTIKEL MIT HAUTPFLEGESALBE AUF DER PERFORIERTEN OBERSTEN SCHICHT
ARTICLE ABSORBANT JETABLE CONTENANT UNE COMPOSITION POUR LE SOIN DE LA PEAU APPLIQUEE SUR UNE FEUILLE SUPERIEURE PERFOREE

(43) Date of publication of application: 06.09.2000
(73) Proprietor: THE PROCTER & GAMBLE COMPANY, Cincinnati, Ohio 45202 (US)
(72) Inventor: ROE, Donald, Carroll, West Chester, OH 45064 (US)
(74) Representative: McGregor, Judit Ester
(86) International application number: PCT/US1997/020842
(87) International publication number: WO 1999/025288

(56) References cited:
- EP-A- 0 040 447
- EP-A- 0 165 807
- EP-A- 0 207 904
- EP-A- 0 272 683
- EP-A- 0 532 005
- EP-A- 0 651 738
- WO- -94//28843
- WO- -96//39109
- WO- -97//05909
- WO-A-95/17867
- DE-A- 4 437 165
- DE-C- 3 309 530
- DE-C1- 3 309 530
- GB-A- 1 498 160
- GB-A- 2 033 751
- GB-A- 2 270 874
- GB-A- 2 311 727
- JP-A- 04 044 762
- US-A- 607 760
- US-A- 3 814 101
- US-A- 3 896 807
- US-A- 5 342 338
- US-A- 5 609 587
- US-A- 5 635 191
- US-A- 5 643 588

## Description

### FIELD OF THE INVENTION

This invention relates to disposable absorbent articles, such as diapers and adult incontinence products, and more particularly to disposable absorbent articles which have the ability to effectively handle both urine and low-viscosity fecal material.

### BACKGROUND OF THE INVENTION

Many types of disposable absorbent products, such as diapers, are available that . have a high capacity for absorbing urine. Disposable products of this type generally comprise some sort of fluid-permeable topsheet material, an absorbent core, and a fluid-impermeable backsheet material. Although these types of absorbent structures may be highly efficient for the absorption of fluids, they cannot absorb bowel movements (i.e., hereinafter referred to as "BM"). Typically, the BM is trapped between the outer surface of the fluid-permeable topsheet and the skin of the wearer, much of it adhering to the wearer's skin.

To prevent BM from adhering to the wearer's skin, the caregiver often applies protective or "repellent" products such as vaseline or mineral oil to the buttocks and anal region before placing the absorbent article on the wearer. This procedure usually involves the caregiver's pouring of the oil or lotion, for example, in one of their hands, rubbing both hands together to distribute the substance thereon and then wiping the same on the skin of the infant. To eliminate the need for this wasteful, messy, and easily forgotten procedure, there have been numerous previous attempts to prepare absorbent articles which contain a protective or therapeutic skin care substance on the topsheet.

One substance that has been applied as a lotion to absorbent products to impart a soothing, protective coating is mineral oil. Mineral oil (also known as liquid petrolatum) is a mixture of various liquid hydrocarbons obtained by distilling the high-boiling (i.e., 300°-390°C) fractions in petroleum. Mineral oil is liquid at ambient temperatures, e.g. 20 °-25°C. As a result, mineral oil is relatively fluid and mobile, even when applied to article topsheets.

Because mineral oil is fluid and mobile at ambient temperatures, it tends not to remain localized on the surface of the topsheet, but instead migrates through the topsheet into the interior of the diaper. Accordingly, relatively high levels of mineral oil need to be applied to the topsheet to provide the desired therapeutic or protective coating lotion benefits. This leads not only to increased costs for these lotioned products, but other detrimental effects as well.

One of these detrimental effects is a decrease in the fluid handling properties as high levels of mineral oil tend to block the topsheet openings. Also, as mineral oil migrates to the interior of the article, it tends to act as a hydrophobic additive, thus decreasing the absorbency of the underlying absorbent core, if one is used. This decrease in absorbency becomes more pronounced as the level of mineral oil applied is increased.

Even without increasing its level, the tendency of mineral oil to migrate once applied has other detrimental effects. For example, the applied mineral oil can transfer to, into and through the packaging or wrapper material for the lotioned product. This can create the need for barrier-type packaging or wrapper films to avoid smearing or other leakage of mineral oil from the product.

To overcome the problems associated with mineral oils, lotions have been applied to absorbent products. Lotioned absorbent products: (1) have therapeutic or protective benefits, (2) do not require relatively high levels of coatings that are liquid at room temperature (e.g., mineral oil); and (3) do not require special wrapping or barrier materials for packaging. Suitable lotions are for example described in US 5 643 588.

While lotioned absorbent products do solve the problems associated with mineral oils, care has to be taken in application of the lotion to the absorbent products to prevent occlusion of the topsheet. Such occlusion of the pervious topsheet prevents urine from penetrating through the topsheet inevitably leading to leakage. In order to overcome this problem, lotion has been applied to topsheets in such a manner so as to not coat the entire surface of the topsheet thereby leaving portions of the topsheet free of lotion. An example of such a coating technique is the application of lotion in stripes spaced apart from one another. While stripes of lotion do permit urine to penetrate the topsheet there is an added complexity in the application process, there is the potential for non-uniform transfer of the lotion to the skin and the potential for contamination of the untreated areas of the topsheet thereby reducing the transfer of urine to the underlying absorbent element.

Therefore, it is an object of the present invention to provide a disposable absorbent article having a structured carrier having superior urine and BM handling properties. As used herein, the term "structured carrier" refers to any two-dimensional or three-dimensional morphological arrangement designed to carry and then transfer a composition while maintaining pathways such that urine may pass therethrough.

It is a further object of the present invention to provide a structured carrier for a comprising a non-woven web disposable absorbent article having an effective open area and a plurality of apertures having a sufficient effective size to allow urine and low-viscosity fecal material penetration therethrough such that a skin care composition can be applied with relative ease as the concern for occlusion of the structured carrier has been removed. Certain nonwoven webs are for example described in DE-A-4 437 165. As used herein, the term "skin care composition" refers to any composition which comprises one or more agents which, when transferred from an article to a wearer's skin, provide a therapeutic and/or protective skin benefit. Representative materials are discussed in detail below.

It is a further object of the present invention to provide an absorbent article having a skin care composition on the outer surface of the structured carrier that is transferable to the wearer's skin and is effective at producing desired skin benefits and/or reducing the adherence of BM to the skin, thereby improving the ease of BM cleanup while not inhibiting the structured carriers ability to handle urine.

These and other objects are obtained using the present invention, as will become readily apparent from a reading of the following disclosure.

### BRIEF SUMMARY OF THE INVENTION

The invention is a disposable absorbent article, such as a diaper. The disposable absorbent article comprises a structured carrier, a liquid impervious backsheet at least partially peripherally joined to the structured carrier, and an absorbent core intermediate the structured carrier and the backsheet. The structured carrier has an inner surface oriented toward the interior of the disposable absorbent article and an outer surface oriented toward the skin of the wearer when the disposable absorbent article is worn. The structured carrier comprises a non-woven web and has an effective open area of at least about 12 percent and a plurality of apertures with an effective size greater than 2.0 square millimeters. The outer surface of the structured carrier comprises an effective amount of a skin care composition which is semi-solid or solid at 20° C and which is partially transferable to the wearer's skin.

### BRIEF DESCRIPTION OF THE DRAWINGS

While the Specification concludes with claims pointing out and distinctly claiming the present invention, it is believed the same will be better understood by the following drawings taken in conjunction with the accompanying Specification wherein like components are given the same reference number and:

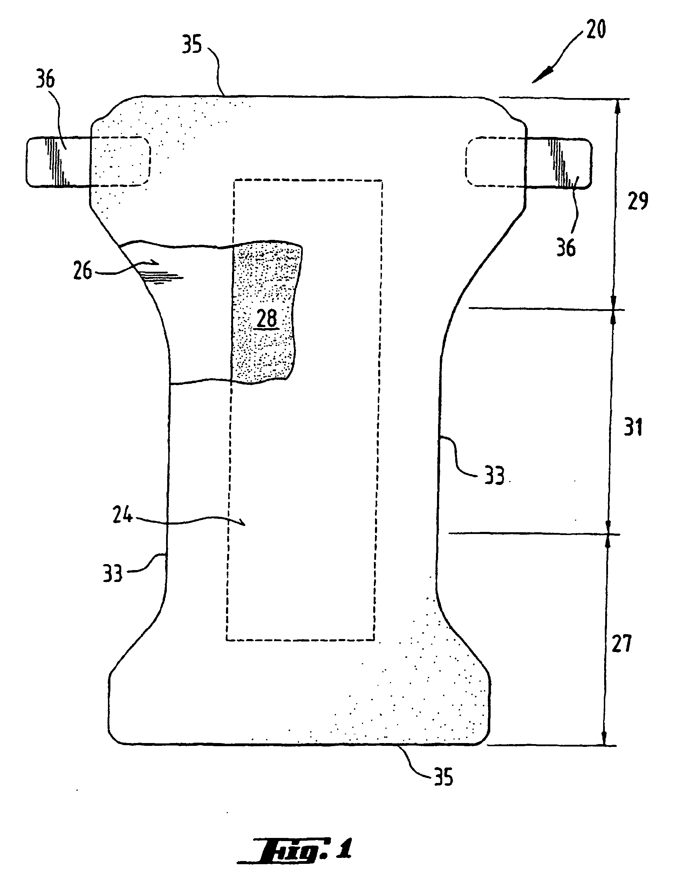

Figure 1 is a top plan view, shown partially in cutaway, of a disposable absorbent article according to the present invention.

### DETAILED DESCRIPTION OF THE INVENTION

As used herein, the term "absorbent article" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use and, preferably, to be recycled, composted or otherwise disposed of in an environmentally compatible manner). A "unitary" absorbent article refers to absorbent articles which are formed of separate parts united together to form a coordinated entity so that they do not require separate manipulative parts like a separate holder and liner. A preferred embodiment of an absorbent article of the present invention is the unitary disposable absorbent article, diaper 20, shown in Figure 1. As used herein, the term "diaper" refers to an absorbent article generally worn by infants and adult incontinent persons and is worn about the lower torso of the wearer. The present invention is also applicable to other absorbent articles such as incontinence briefs, incontinence undergarments, absorbent inserts, diapers holders and liners, feminine hygiene garments, and the like.

Figure 1 is a plan view of the diaper 20 of the present invention in its flat-out, uncontracted state (i.e., with elastic induced contraction pulled out) with portions of the structure being cut-away to more clearly show the construction of the diaper 20 and with the portion of the diaper 20 which faces or contacts the wearer, the inner surface, oriented towards the viewer. As shown in Figure 1, the diaper 20 preferably comprises a liquid pervious structured carrier 24; a liquid impervious backsheet 26 joined with the structured carrier 24; and an absorbent core 28 intermediate the structured carrier 24 and the backsheet 26. The diaper 20 may further comprise elasticized side panels (not shown); elasticized leg cuffs (not shown); an elastic waist feature (not shown); and a fastening system with tape tabs generally multiply designated as 36.

The diaper 20 is shown in Figure 1 to have a first waist region 27 juxtaposed with the front of the wearer while the diaper 20 is being worn, a second waist region 29 opposed to the first waist region 27 and juxtaposed with the back of the wearer while the diaper 20 is being worn, a crotch region 31 positioned between the first waist region 27 and the second waist region 29, and a periphery which is defined by the outer edges of the diaper 20 in which the longitudinal edges are designated 33 and the end edges are designated 35. The inner surface of the diaper 20 comprises that portion of the diaper 20 which is adjacent to the wearer's body during use (i.e., the inner surface generally is formed by at least a portion of the structured carrier 24 and other components joined to the structured carrier 24). The outer surface comprises that portion of the diaper 20 which is positioned away from the wearer's body (i.e., the outer surface generally is formed by at least a portion of the backsheet 26 and other components joined to the backsheet 26) during use.

Figure 1 shows an embodiment of the diaper 20 in which the structured carrier 24 and the backsheet 26 have length and width dimensions generally larger than those of the absorbent core 28. The structured carrier 24 and the backsheet 26 extend beyond the edges of the absorbent core 28 to thereby form the periphery of the diaper 20. While the structured carrier 24, the backsheet 26, and the core 28 may be assembled in a variety of well known configurations, preferred diaper configurations are described generally in U.S. Patent 3,860,003 entitled "Contactable Side Portions for Disposable Diaper" which issued to Kenneth B. Buell on January 14, 1975; and U.S. Patent 5,151,092, "Absorbent Article With Dynamic Elastic Waist Feature Having A Predisposed Resilient Flexural Hinge", issued to Kenneth B. Buell et al. September 29, 1992.

The absorbent core 28 may be any absorbent means which is generally compressible, conformable, non-irritating to the wearer's skin, and capable of absorbing and retaining liquids such as urine and other certain body exudates. As shown in Figure 1, the absorbent core 28 has a garment surface, a body surface, side edges, and waist edges. The absorbent core 28 may be manufactured in a wide variety of sizes and shapes (e.g., rectangular, hourglass, "T"-shaped, asymmetric, etc.) and from a wide variety of liquid-absorbent materials commonly used in disposable diapers and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials.

The configuration and construction of the absorbent core 28 may also be varied (e.g., the absorbent core 28 may have varying caliper zones, a hydrophilic gradient, a superabsorbent gradient, or lower average density and lower average basis weight acquisition zones; or may comprise one or more layers or structures). The total absorbent capacity of the absorbent core 28 should, however, be compatible with the design loading and the intended use of the diaper 20. Further, the size and absorbent capacity of the absorbent core 28 may be varied to accommodate wearers ranging from infants through adults.

Exemplary absorbent structures for use as the absorbent core 28 are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,888,231 entitled "Absorbent Core Having A Dusting Layer" issued to Angstadt on December 19, 1989; and U.S. Patent 4,834,735, entitled "High Density Absorbent Members Having Lower Density and Lower Basis Weight Acquisition Zones", issued to Alemany et al. on May 30, 1989.

The backsheet 26 is positioned adjacent the garment surface of the absorbent core 28 and is preferably joined thereto by attachment means (not shown) such as those well known in the art. As used herein, the term "joined" encompasses configurations whereby an element is directly secured to the other element by affixing the element directly to the other element, and configurations whereby the element is indirectly secured to the other element by affixing the element to intermediate member(s) which in turn are affixed to the other element.

For example, the backsheet 26 may be secured to the absorbent core 28 by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota and marketed as HL-1258. The attachment means will preferably comprise an open pattern network of filaments of adhesive as is disclosed in U.S. Patent 4,573,986 entitled "Disposable Waste-Containment Garment", which issued to Minetola et al. on March 4, 1986, more preferably several lines of adhesive filaments swirled into a spiral pattern such as is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Alternatively, the attachment means may comprise heat bonds, pressure bonds, ultrasonic bonds, dynamic mechanical bonds, or any other suitable attachment means or combinations of these attachment means as are known in the art.

The backsheet 26 is impervious to liquids (e.g., urine) and is preferably manufactured from a thin plastic film, although other flexible liquid impervious materials may also be used. As used herein, the term "flexible" refers to materials which are compliant and will readily conform to the general shape and contours of the human body.

The backsheet 26 prevents the exudates absorbed and contained in the absorbent core 28 from wetting articles which contact the diaper 20 such as bedsheets and undergarments. The backsheet 26 may thus comprise a woven or nonwoven material, polymeric films such as thermoplastic films of polyethylene or polypropylene, or composite materials such as a film-coated nonwoven material. Preferably, the backsheet 26 is a thermoplastic film having a thickness of about 0.012 mm (0.5 mil) to about 0.051 mm (2.0 mils). Particularly preferred materials for the backsheet 26 include RR8220 blown films and RR5475 cast films as manufactured by Tredegar Industries, Inc. of Terre Haute, Indiana. The backsheet 26 is preferably embossed and/or matte finished to provide a more clothlike appearance. Further, the backsheet 26 may permit vapors to escape from the absorbent core 28 (i.e., be breathable) while still preventing exudates from passing through the backsheet 26.

The structured carrier 24 has a first or inner surface oriented toward the interior of the disposable diaper, specifically oriented toward the absorbent core 28, and an opposed second or outer surface oriented toward the skin of the wearer when the diaper is worn.

The structured carrier 24 is juxtaposed with, but not necessarily adjacent the body surface of the absorbent core 28, and is preferably joined to the backsheet 26 or absorbent core 28 by means such as those well known in the art. Suitable attachment means are described above with respect to joining the backsheet 26 to the absorbent core 28. In a preferred embodiment of the present invention, the structured carrier 24 and the backsheet 26 are joined directly to each other in the diaper periphery.

The structured carrier 24 is compliant, soft feeling, and non-irritating to the wearer's skin. Further, the structured carrier 24 is liquid pervious, permitting liquids (e.g., urine) to readily penetrate through its thickness. A suitable structured carrier 24 is nonwoven webs of natural fibers (e.g., wood or cotton fibers), synthetic fibers (e.g., polyester or polypropylene fibers), or a combination of natural and synthetic fibers. Preferably, the structured carrier 24 is made of a hydrophobic material to isolate the wearer's skin from liquids contained in the absorbent core 28. Alternatively, the structured carrier 24 may be surfactant treated to make it hydrophilic.

The structured carrier 24 preferably has a plurality of apertures with an effective aperture size of at least 2.0 square millimeters. Effective apertures are those which have a gray level of 18 or less on a standard gray level scale of 0-255, under the image acquisition parameters described below.

The structured carrier 24 preferably has an effective open area of at least 15 percent, more preferably the structured carrier has an effective open area of at least 20 percent, even more preferably, the structured carrier has an effective open area of at least 25 percent, and most preferably the structured carrier has an effective open area of at least 30 percent.

The effective aperture size and effective open area are determined by the following procedure using the image analysis described below. The procedure has three principal steps: image acquisition, i.e., obtaining representative images of areas on the surface of the structured carrier 24; image measurement, i.e., measuring the percentage open area of an image and of individual apertures and their perimeters; and data analysis, i.e., exporting the percentage open area, individual aperture area, and perimeter measurements to a spreadsheet where frequency distributions, sum of area distributions, and hydraulic radius computations are made.

An image analysis system having a frame grabber board, microscope, camera and image analysis software is utilized. A model DT2855 frame grabber board available from Data Translation of Marlboro, Mass. is provided. A VH5900 monitor microscope, a video camera, having aVH50 lens with a contact type illumination head available from the Keyence Company of Fair Lawn, N.J. are also provided and used to acquire an image to be saved to computer file. The Keyence microscope acquires the image and the frame grabber board converts the analog signal of this image into computer readable digital format. The image is saved to computer file and measured using suitable software such as the Optimas Image Analysis software, version 3.1, available from the BioScan Company of Edmaons, Wash. In order to use the Optimas Image Analysis software, the computer should have Windows software, version 3.0 or later, available from the Microsoft Corporation of Redmond, Wash. And also have a CPU at least equivalent to the Intel 80386. Any suitable desk top PC may be used, with a 486 DX33 type PC having been found to be particularly suitable. Images being saved to and recalled from file were displayed on a Sony Trinitron monitor model PVM-1343MO with a final display magnification of about 50X.

The image acquisition step, noted above requires 10 different regions from a representative structured carrier 24 sample of a particular type of diaper 20 or from sample material to be tested. Each region is rectangular, measuring about 5.8 millimeters by 4.2 millimeters. The sample is placed on a black mat board to increase the contrast between the apertures and the portion of the sample which defines the apertures. The mean gray level and standard deviation of the black mat board were 16 and 4, respectively.

Images are acquired with room lights off using the Keyence monitor microscope mounted on a copystand directly above the sample. The Keyence light source illuminating the sample is adjusted and monitored with the Optimas software to measure the mean gray level and standard deviation of a 0.3 density wedge on a Kodak Gray Scale available from Eastman Kodak Company of Rochester, New York. The control of Keyence light source is adjusted so that the mean gray level of the illuminated wedge is 111 ± 1 and the standard deviation is 10 ± 1. All images were acquired during a single time period, and the Keyence light source is monitored by measuring the mean gray level and standard deviation of the wedge throughout the image acquisition process.

In measuring an individual aperture, only the effective aperture size is of interest. Measuring the effective aperture size quantifies the aperture size intended to contribute to the porosity of the structured carrier 24, and account for contributions of fibers and fiber bundles which traverse an area intended to be an aperture. An effective aperture is any hole through the structured carrier 24 having a gray level less than or equal to 18 using image acquisition parameters as described herein. Thus, an intended aperture may be divided into plural effective apertures by traverse fibers.

The image analysis software is calibrated in millimeters by a ruler image acquired from the sample images. A 3 by 3 pixel averaging filter found in the Optimas 3.1 Image menu is applied to each saved image to reduce noise. The apertures are detected in the gray level range of 0 through 18. An aperture which is not fully contained within the 5.8 by 4.2 viewing area is not considered in the individual area and perimeter measurements. Therefore, area and perimeter averages and distributions are not affected by apertures which are not wholly contained within the field of view.

However, individual apertures which could not be fully viewed in the image are included in the percentage open area calculation. This difference occurs because the percent open area is simply the image of pixel ratios from 0 through 18 to the total number of pixels in the image. Areas having a gray level 19 or greater were not counted in the open area calculation.

The percentage open area for the average of 10 images for each structured carrier 24 is measured using the Optimas Image Analysis software. The percentage open area is defined as the ratio of the number of pixels having a gray level from 0 through 18 to the total number of pixels for the image. The percentage open area is measured for each image representing one particular region from a structured carrier sample. The percentage open area from each of the 10 individual images is then averaged to yield a percentage open area for the entire sample.

The data analysis is conducted by an Excel spreadsheet, also available from the Microsoft Corporation of Redmond, Washington. The Excel spreadsheet organized the percentage open area, aperture area, and aperture perimeter measurements obtained from the Optimas software. Sample averages and standard deviations, size and frequency distributions of individual aperture areas and hydraulic radius computations (area divided by perimeter) for individual apertures are obtained using the spreadsheet.

Distributions of individual aperture area are also computed using the Excel spreadsheet. The apertures are sorted into bins of certain size ranges. The number of aperture areas falling into certain size ranges of interest is determined as well as the sum of the areas within each range. The ranges are set in increments of 0.05 square millimeters. These areas are expressed as a percentage of the total open area of the sample. The frequency and sum of the area distributions are obtained by combining individual aperture measurements from all 10 images for each sample.

There are a number of manufacturing techniques which may be used to manufacture the structured carrier 24. For example, the structured carrier 24 may be a nonwoven web of fibers spunbonded, carded, wet-laid, meltblown, hydroentangled, combinations or composite laminates of the above, or the like. Preferred structured carriers 24 include a carded/carded composite, hydroentangled over a wire forming screen and thermally air-through bonded by means well known to those skilled in the nonwovens art and hydroentanglement of fibrous webs. Alternatively, apertured formed films, woven netting, and woven apertured netting may be suitable.

While the specific composition(s) delivered (referred to herein as "skin care composition" and "composition") in accordance with the present method is not the critical factor in achieving improved skin condition of the area under the absorbent article, it is apparent that the composition must provide either a protective, nonocclusive function (e.g., a relatively liquid impervious but vapor pervious barrier) to avoid skin hyperhydration and skin exposure to materials contained in body exudates, or it must contain agents that deliver, either directly or indirectly. skin care benefits. For example, indirect benefits include improved removal of skin irritants such as feces or urine. The composition may be in a variety of forms, including, but not limited to, emulsions, lotions, creams, ointments, salves, powders, suspensions, encapsulations, gels, and the like.

As used herein, the term "effective amount of a skin care composition" refers to an amount of a particular composition which, when applied or migrated to one or more of the wearer-contacting surface(s) of an absorbent article(s), will be effective in providing a protective barrier and/or delivering a skin care benefit when delivered via absorbent articles over time. Of course, the effective amount of composition applied to the article will depend, to a large extent, on the particular composition used. Nonetheless, the quantity of the composition on at least a portion of the wearer-contacting surface of the absorbent article will preferably range from about 0.05 mg/in² (0.0078 mg/cm²) to about 80 mg/in² (12.4 mg/cm²), more preferably from about 1 mg/in² (0.16 mg/cm²) to about 40 mg/in² (6.20 mg/cm²), still more preferably from about 4 mg/in² (0.62 mg/cm²) to about 26 mg/in² (4.03mg/cm²). These ranges are by way of illustration only and the skilled artisan will recognize that the nature of the composition will dictate the level that must be applied to achieve the desired skin benefits, and that such levels are ascertainable by routine experimentation in light of the present disclosure.

While the level of skin care composition applied to the absorbent article is an important aspect of the present methods, more important is the amount of composition transferred to the wearer's skin during use of one or more treated articles. Though the requisite level delivered to the skin to provide the desired skin benefits will depend to some degree on the nature of the composition employed, Applicants have found that relatively low levels may be delivered while still providing the desired skin effects. This is particularly true for preferred compositions.

Another benefit of the present method is the controlled application of the skin care composition to deliver the low but effective levels of composition required. This is in contrast to typically sporadic manual application of skin care agents, where the caregiver/user often applies significantly greater levels of material than are needed. Excessive materials added manually may adversely impact the fluid handling properties of the absorbent article, as a result of transfer from the skin to the article. Indeed, for certain materials, such as petrolatum, the levels applied manually may actually result in an occlusive effect, thereby compromising the skin. A benefit of the present methods is providing a barrier to surface moisture while avoiding occlusion of the skin (i.e., maintaining skin breathability). Thus, the present methods, which allow controlled composition delivery throughout the wear period, allow transfer of optimal levels of the composition to the skin to improve skin condition.

With regard to the level of skin care composition that is transferred to the wearer during use of one treated absorbent article worn for a period of about 3 hours (a typical daytime wear time), preferred is where at least about 0.01 mg/in² (0.0016 mg/cm²), more preferably at least about 0.05 mg/in² (0.0078 mg/cm²), still more preferably at least about 0.1 mg/in² (0.016 mg/cm²), of the composition is transferred to the skin over a three hour wear period. Typically, the amount of composition delivered by one treated article will be from about 0.01 mg/in² (0.0016 mg/cm²) to about 5 mg/in² (0.78 mg/cm²), more preferably from about 0.05 mg/in² (0.0078 mg/cm²) to about 3 mg/in² (0.47 mg/cm²), still more preferably from about 0.1 mg/in² (0.016 mg/cm²) to about 2 mg/in² (0.31 mg/cm²), over a three hour wear period.

For continual use of treated articles (in other words, changes occur in accordance with normal use patterns, which typically include changes every 3 to 4 hours during the day and a fresh article before overnight sleep) such as for a period of 24 hours, it will be preferred that at least about 0.03 mg/in² (0.0047 mg/cm²), more preferably at least about 0.1 mg/in² (0.016 mg/cm²), still more preferably at least about 0.3 mg/in² (0.047 mg/cm²), of the composition is transferred to the wearer's skin over the 24 hour period. Typically, the amount of composition delivered after a period of 24 hours where treated articles are applied at each change, will be from about 0.03 mg/in² (0.0047 mg/cm²) to about 18 mg/in² (2.79 mg/cm²), more typically from about 0.1 mg/in² (0.016 mg/cm²) to about 10 mg/in² (1.55 mg/cm²), still more typically from about 0.3 mg/in² (0.047 mg/cm²) to about 6 mg/in² (0.93 mg/cm²).

It will be recognized that of the numerous materials useful in the skin care compositions delivered to skin in accordance with the present methods, those that have been deemed safe and effective skin care agents are logical materials for use herein. Such materials include Category I actives as defined by the U.S. Federal Food and Drug Administration's (FDA) Tentative Final Monograph on Skin Protectant Drug Products for Over-the-Counter Human Use, which presently include: alantoin, aluminum hydroxide gel, calamine, cocoa butter, dimethicone, cod liver oil (in combination), glycerine, kaolin, petrolatum, lanolin, mineral oil, shark liver oil, white petrolatum, talc, topical starch, zinc acetate, zinc carbonate, zinc oxide, and the like. Other potentially useful materials are Category III actives as defined by the U.S. Federal Food and Drug Administration's Tentative Final Monograph on Skin Protectant Drug Products for Over-the-Counter Human Use tentative final monograph on skin protectant drug products for over-the-counter human use, which presently include: live yeast cell derivatives, aldioxa, aluminum acetate, microporous cellulose, cholecalciferol, colloidal oatmeal, cysteine hydrochloride, dexpanthanol, Peruvian balsam oil, protein hydrolysates, racemethionine, sodium bicarbonate, Vitamin A, and the like.

Many of the FDA monographed skin care ingredients are currently utilized in commercially available skin care products, such as A and D® Ointment, Vaseline® Petroleum Jelly, Desitin® Diaper Rash Ointment and Daily Care Ointment, Gold Bond® Medicated Baby Powder, Aquaphor® Healing Ointment, Baby Magic® Baby Lotion, Johnson's Ultra Sensitive® Baby Cream, Johnson's baby lotion, lip balms, etc. These commercial products may be applied to absorbent articles to create treated articles for use in the present methods, either with or without modification of the product to facilitate delivery via this novel method.

As will be discussed hereinafter, the skin care compositions useful in the methods of the present invention preferably, though not necessarily, have a melting profile such that they are relatively immobile and localized on the wearer-contacting surface of the article at room temperature, are readily transferable to the wearer at body temperature, and yet are not completely liquid under extreme storage conditions. Preferably, the compositions are easily transferable to the skin by way of normal contact, wearer motion, and/or body heat. Because the composition preferably is substantially immobilized on the article's wearer-contacting surface, relatively low levels of composition are needed to impart the desired skin care benefits. In addition, special barrier or wrapping materials may be unnecessary in packaging the treated articles useful in the methods of the present invention.

In a preferred embodiment, the skin care compositions useful herein are solid, or more often semi-solid, at 20°C, i.e. at ambient temperatures. By "semisolid" is meant that the composition has a rheology typical of pseudoplastic or plastic liquids. When no shear is applied, the compositions can have the appearance of a semi-solid but can be made to flow as the shear rate is increased. This is due to the fact that, while the composition contains primarily solid components, it also includes some minor liquid components. Preferably, the compositions of the present invention have a zero shear viscosity between about 1.0 X 10⁶ centipoise and about 1.0 X 10⁸. More preferably, the zero shear viscosity is between about 5.0 X 10⁶ centipoise and about 5.0 X 10⁷ centipoise. As used herein the term "zero shear viscosity" refers to a viscosity measured at very low shear rates (e.g., 1.0 sec⁻¹) using plate and cone viscometer (a suitable instrument is available from TA Instruments of New Castle, DE as model number CSL 100). One of skill in the art will recognize means other than high melting point components (as discussed below) can be used to provide comparable viscosities measured for such compositions comprising such means can be measured by extrapolating a plot of viscosity vs. shear rate for such compositions to a shear rate of zero at a temperature of about 20°C.

Preferred compositions are at least semi-solid at room temperature to minimize composition migration. In addition, the compositions preferably have a final melting point (100% liquid) above potential "stressful" storage conditions that can be greater than 45°C (e.g., warehouse in Arizona, car trunk in Florida, etc.). Representative compositions having these melt characteristics are described in detail in U.S. Patent No. 5,643,588 (Roe et al.), U.S. Patent No. 5,607,760 (Roe et al.), U.S. Patent No. 5,609,587, and U.S. Patent No. 5,635,191. Specifically, preferred compositions will have the following melt profile:

| Characteristic | Preferred Range | Most Preferred |
|---|---|---|
| % liquid at room temp. (20°C) | 2-50 | 3-25 |
| % liquid at body temp. (37°C) | 25-95 | 30-90 |
| final melting point (°C) | ≥38 | ≥45 |

By being solid or semisolid at ambient temperatures, preferred compositions do not have a tendency to flow and migrate to a significant degree to undesired locations of the article to which they are applied. This means less skin care composition is required for imparting desirable therapeutic, protective or conditioning benefits.

To enhance immobility of preferred compositions, the viscosity of the formulated compositions should be as high as possible to prevent flow within the article to undesired location. Unfortunately, in some instances, higher viscosities may inhibit transfer of composition to the wearer's skin. Therefore, a balance should be achieved so the viscosities are high enough to keep the compositions localized on the surface of the article, but not so high as to impede transfer to the wearer's skin. Suitable viscosities for the compositions will typically range from about 5 to about 500 centipoise, preferably from about 5 to about 300 centipoise, more preferably from about 5 to about 100 centipoise, measured at 60_C using a rotational viscometer (a suitable viscometer is available from Lab Line Instruments, Inc. of Melrose Park, IL as Model 4537). The viscometer is operated at 60 rpm using a number 2 spindle.

For compositions designed to provide a skin smoothness benefit, a useful active ingredient in these compositions is one or more skin protectants or emollients. As used herein, the term "emollient" is a material that protects against wetness or irritation, softens, soothes, supples, coats, lubricates, moisturizes, protects and/or cleanses the skin. (It will be recognized that several of the monographed actives listed above are "emollients", as that term is used herein.) In a preferred embodiment, these emollients will have either a plastic or liquid consistency at ambient temperatures, i.e., 20°C. This particular emollient consistency allows the composition to impart a soft, lubricious, lotion-like feel.

Representative emollients useful in the present invention include, but are not limited to, emollients that are petroleum-based; polyol polyesters; sucrose ester fatty acids; polyethylene glycol and derivatives thereof; humectants; fatty acid ester type; alkyl ethoxylate type; fatty acid ester ethoxylates; fatty alcohol type; polysiloxane type; propylene glycol and derivatives thereof; glycerine and derivatives thereof, including glyceride, acetoglycerides, and ethoxylated glycerides of C₁₂-C₂₈ fatty acids; triethylene glycol and derivatives thereof; spermaceti or other waxes; fatty acids; fatty alcohol ethers, particularly those having from 12 to 28 carbon atoms in their fatty chain, such as stearic acid; propoxylated fatty alcohols; other fatty esters of polyhydroxy alcohols; lanolin and its derivatives; kaolin and its derivatives; any of the monographed skin care agents listed above; or mixtures of these emollients. Suitable petroleum-based emollients include those hydrocarbons, or mixtures of hydrocarbons, having chain lengths of from 16 to 32 carbon atoms. Petroleum based hydrocarbons having these chain lengths include mineral oil (also known as "liquid petrolatum") and petrolatum (also known as "mineral wax," "petroleum jelly" and "mineral jelly"). Mineral oil usually refers to less viscous mixtures of hydrocarbons having from 16 to 20 carbon atoms. Petrolatum usually refers to more viscous mixtures of hydrocarbons having from 16 to 32 carbon atoms. Petrolatum and mineral oil are particularly preferred emollients for compositions of the present invention.

Suitable fatty acid ester type emollients include those derived from C₁₂-C₂₈ fatty acids, preferably C₁₆-C₂₂ saturated fatty acids, and short chain (C₁-C₈, preferably C₁-C₃) monohydric alcohols. Representative examples of such esters include methyl palmitate, methyl stearate, isopropyl laurate, isopropyl myristate, isopropyl palmitate, ethylhexyl palmitate and mixtures thereof. Suitable fatty acid ester emollients can also be derived from esters of longer chain fatty alcohols (C₁₂-C₂₈, preferably C₁₂-C₁₆) and shorter chain fatty acids e.g., lactic acid, such as lauryl lactate and cetyl lactate.

Suitable alkyl ethoxylate type emollients include C₁₂-C₂₂ fatty alcohol ethoxylates having an average degree of ethoxylation of from about 2 to about 30. Preferably, the fatty alcohol ethoxylate emollient is selected from the group consisting of lauryl, cetyl, and stearyl ethoxylates, and mixtures thereof, having an average degree of ethoxylation ranging from about 2 to about 23. Representative examples of such alkyl ethoxylates include laureth-3 (a lauryl ethoxylate having an average degree of ethoxylation of 3), laureth-23 (a lauryl ethoxylate having an average degree of ethoxylation of 23), ceteth-10 (a cetyl alcohol ethoxylate having an average degree of ethoxylation of 10) and steareth-10 (a stearyl alcohol ethoxylate having an average degree of ethoxylation of 10). when employed, these alkyl ethoxylate emollients are typically used in combination with the petroleum-based emollients, such as petrolatum, at a weight ratio of alkyl ethoxylate emollient to petroleum-based emollient of from about 1:1 to about 1:5, preferably from about 1:2 to about 1:4.

Suitable fatty alcohol type emollients include C₁₂-C₂₂ fatty alcohols, preferably C₁₆-C₁₈ fatty alcohols. Representative examples include cetyl alcohol and stearyl alcohol, and mixtures thereof. When employed, these fatty alcohol emollients are typically used in combination with the petroleum-based emollients, such as petrolatum, at a weight ratio of fatty alcohol emollient to petroleum-based emollient of from about 1:1 to about 1:5, preferably from about 1:1 to about 1:2.

Other suitable types of emollients for use herein include polysiloxane compounds. In general, suitable polysiloxane materials for use in the present invention include those having monomeric siloxane units of the following structure: 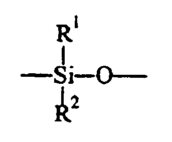 wherein, R¹ and R², for each independent siloxane monomeric unit can each independently be hydrogen or any alkyl, aryl, alkenyl, alkaryl, arakyl, cycloalkyl, halogenated hydrocarbon, or other radical. Any of such radicals can be substituted or unsubstituted. R¹ and R² radicals of any particular monomeric unit may differ from the corresponding functionalities of the next adjoining monomeric unit. Additionally, the polysiloxane can be either a straight chain, a branched chain or have a cyclic structure. The radicals R¹ and R² can additionally independently be other silaceous functionalities such as, but not limited to siloxanes, polysiloxanes, silanes, and polysilanes. The radicals R¹ and R² may contain any of a variety of organic functionalities including, for example, alcohol, carboxylic acid, phenyl, and amine functionalities.

Exemplary alkyl radicals are methyl, ethyl, propyl, butyl, pentyl, hexyl, octyl, decyl, octadecyl, and the like. Exemplary alkenyl radicals are vinyl, allyl, and the like. Exemplary aryl radicals are phenyl, diphenyl, naphthyl, and the like. Exemplary alkaryl radicals are toyl, xylyl, ethylphenyl, and the like. Exemplary aralkyl radicals are benzyl, alpha-phenylethyl, beta-phenylethyl, alpha-phenylbutyl, and the like. Exemplary cycloalkyl radicals are cyclobutyl, cyclopentyl, cyclohexyl, and the like. Exemplary halogenated hydrocarbon radicals are chloromethyl, bromoethyl, tetrafluorethyl, fluorethyl, trifluorethyl, trifluorotloyl, hexafluoroxylyl, and the like.

Viscosity of polysiloxanes useful may vary as widely as the viscosity of polysiloxanes in general vary, so long as the polysiloxane is flowable or can be made to be flowable for application to the article. This includes, but is not limited to, viscosity as low as 5 centistokes (at 37_C as measured by a glass viscometer) to about 20,000,000 centistokes. Preferably the polysiloxanes have a viscosity at 37°C ranging from about 5 to about 5,000 centistokes, more preferably from about 5 to about 2,000 centistokes, most preferably from about 100 to about 1000 centistokes. High viscosity polysiloxanes which themselves are resistant to flowing can be effectively deposited upon the article by such methods as, for example, emulsifying the polysiloxane in surfactant or providing the polysiloxane in solution with the aid of a solvent, such as hexane, listed for exemplary purposes only. Particular methods for applying polysiloxane emollients to absorbent articles are discussed in more detail hereinafter.

Preferred polysiloxanes compounds for use in the present invention are disclosed in U.S. Patent 5,059,282 (Ampulski et al), issued October 22, 1991. Particularly preferred polysiloxane compounds for use as emollients in the compositions of the present invention include phenyl-functional polymethylsiloxane compounds (e.g., Dow Corning 556 Cosmetic-Grade Fluid: polyphenylmethylsiloxane) and cetyl or stearyl functionalized dimethicones such as Dow 2502 and Dow 2503 polysiloxane liquids, respectively. In addition to such substitution with phenyl-functional or alkyl groups, effective substitution may be made with amino, carboxyl, hydroxyl, ether, polyether, aldehyde, ketone, amide, ester, and thiol groups. Of these effective substituent groups, the family of groups comprising phenyl, amino, alkyl, carboxyl, and hydroxyl groups are more preferred than the others; and phenyl-functional groups are most preferred.

Suitable humectants include glycerine, propylene glycol, sorbitol, trihydroxy stearin, and the like.

When present, the amount of emollient that can be included in the composition will depend on a variety of factors, including the particular emollient involved, the lotion-like benefits desired, the other components in the composition and like factors. The composition will comprise from 0 to about 100%, by total weight, of the emollient. Preferably, the composition will comprise from about 10 to about 95%, more preferably from about 20 to about 80%, and most preferably from about 40 to about 75%, by weight, of the emollient.

Another optional, preferred component of the therapeutic/skin protective/skin conditioning compositions useful in the methods of the present invention is an agent capable of immobilizing the composition (including the preferred emollient and/or other skin conditioning/therapeutic/protective agents) in the desired location in or on the treated article. Because certain of the preferred emollients in the composition have a plastic or liquid consistency at 20°C, they tend to flow or migrate, even when subjected to modest shear. When applied to a wearer-contacting surface or other location of an absorbent article, especially in a melted or molten state, the emollient will not remain primarily in or on the treated region. Instead, the emollient will tend to migrate and flow to undesired regions of the article.

Specifically, if the emollient migrates into the interior of the article, it can cause undesired effects on the absorbency of the article core due to the hydrophobic characteristics of many of the emollients and other skin conditioning agents used in the compositions useful in the methods of the present invention. It also means that much more emollient has to be applied to the article to get the desired skin smoothness benefits. Increasing the level of emollient not only increases the cost, but also exacerbates the undesirable effect on the absorbency of the article's core and undesired transfer of composition during processing/converting of the treated articles.

The immobilizing agent counteracts this tendency of the emollient to migrate or flow by keeping the emollient primarily localized on the surface or in the region of the article to which the composition is applied. This is believed to be due, in part, to the fact that the immobilizing agent raises the melting point and/or viscosity of the composition above that of the emollient. Since the immobilizing agent is preferably miscible with the emollient (or solubilized in the emollient with the aid of an appropriate emulsifier or dispersed therein), it entraps the emollient on the surface of the article's wearer contacting surface or in the region to which it is applied.

It is also advantageous to "lock" the immobilizing agent on the wearer contacting surface or the region of the article to which it is applied. This can be accomplished by using immobilizing agents which quickly set up (i.e., solidify) upon application to the article. In addition, outside cooling of the treated article via blowers, fans, cold rolls, etc. can speed up crystallization of the immobilizing agent.

In addition to being miscible with (or solubilized in) the emollient, the immobilizing agent will preferably have a melting profile that will provide a composition that is solid or semisolid at ambient temperature. In this regard, preferred immobilizing agents will have a melting point of at least about 35°C. This is so the immobilizing agent itself will not have a tendency to migrate or flow. Preferred immobilizing agents will have melting points of at least about 40°C. Typically, the immobilizing agent will have a melting point in the range of from about 50_ to about 150_C.

When utilized, immobilizing agents useful herein can be selected from any of a number of agents, so long as the preferred properties of the skin care composition provide the skin benefits described herein. Preferred immobilizing agents will comprise a member selected from the group consisting of C₁₄-C₂₂ fatty alcohols, C₁₂-C₂₂ fatty acids, and C₁₂-C₂₂ fatty alcohol ethoxylates having an average degree of ethoxylation ranging from 2 to about 30, and mixtures thereof. Preferred immobilizing agents include C₁₆-C₁₈ fatty alcohols, most preferably crystalline high melting materials selected from the group consisting of cetyl alcohol, stearyl alcohol, behenyl alcohol, and mixtures thereof. (The linear structure of these materials can speed up solidification on the treated absorbent article.) Mixtures of cetyl alcohol and stearyl alcohol are particularly preferred. Other preferred immobilizing agents include C₁₆-C₁₈ fatty acids, most preferably selected from the group consisting of palmitic acid, stearic acid, and mixtures thereof. Mixtures of palmitic acid and stearic acid are particularly preferred. Still other preferred immobilizing agents include C₁₆-C₁₈ fatty alcohol ethoxylates having an average degree of ethoxylation ranging from about 5 to about 20. Preferably, the fatty alcohols, fatty acids and fatty alcohols are linear. Importantly, these preferred immobilizing agents such as the C₁₆ - C₁₈ fatty alcohols increase the rate of crystallization of the composition causing the composition to crystallize rapidly onto the surface of the substrate.

Other types of ingredients that can be used as immobilizing agents, either alone, or in combination with the above-mentioned immobilizing agents, include waxes such as carnauba, ozokerite, beeswax, candelilla, paraffin, ceresin, esparto, ouricuri, rezowax, isoparaffin, and other known mined and mineral waxes. The high melt point of these materials can help immobilize the composition on the desired surface or location on the article. Additionally microcrystalline waxes are effective immobilizing agents. Microcrystalline waxes can aid in "locking" up low molecular weight hydrocarbons within the skin care composition. Preferably the wax is a paraffin wax. An example of a particularly preferred alternate immobilizing agent is a paraffin wax such as Parrafin S.P. 434 from Strahl and Pitsch Inc. P.O. Box 1098 West Babylon, NY 11704.

Suitable polyhydroxy fatty acid esters for use in the present invention will have the formula: 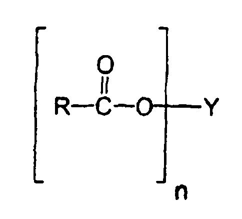 wherein R is a C₅-C₃₁ hydrocarbyl group, preferably straight chain C₇-C₁₉ alkyl or alkenyl, more preferably straight chain C₉-C₁₇ alkyl or alkenyl, most preferably straight chain C₁₁-C₁₇ alkyl or alkenyl, or mixture thereof; Y is a polyhydroxyhydrocarbyl moiety having a hydrocarbyl chain with at least 2 free hydroxyls directly connected to the chain; and n is at least 1. Suitable Y groups can be derived from polyols such as glycerol, pentaerythritol; sugars such as raffinose, maltodextrose, galactose, sucrose, glucose, xylose, fructose, maltose, lactose, mannose and erythrose; sugar alcohols such as erythritol, xylitol, malitol, mannitol and sorbitol; and anhydrides of sugar alcohols such as sorbitan.

One class of suitable polyhydroxy fatty acid esters for use in the present invention comprises certain sorbitan esters, preferably the sorbitan esters of C₁₆-C₂₂ saturated fatty acids. Because of the manner in which they are typically manufactured, these sorbitan esters usually comprise mixtures of mono-, di-, tri-, etc. esters. Representative examples of suitable sorbitan esters include sorbitan palmitates (e.g., SPAN 40), sorbitan stearates (e.g., SPAN 60), and sorbitan behenates, that comprise one or more of the mono-, di- and tri-ester versions of these sorbitan esters, e.g., sorbitan mono-, di- and tri-palmitate, sorbitan mono-, di- and tri-stearate, sorbitan mono-, di and tri-behenate, as well as mixed tallow fatty acid sorbitan mono-, di- and tri-esters. Mixtures of different sorbitan esters can also be used, such as sorbitan palmitates with sorbitan stearates. Particularly preferred sorbitan esters are the sorbitan stearates, typically as a mixture of mono-, di- and tri-esters (plus some tetraester) such as SPAN 60, and sorbitan stearates sold under the trade name GLYCOMUL-S by Lonza, Inc. Although these sorbitan esters typically contain mixtures of mono-, di- and tri-esters, plus some tetraester, the mono- and di-esters are usually the predominant species in these mixtures.

Another class of suitable polyhydroxy fatty acid esters for use in the present invention comprises certain glyceryl monoesters, preferably glyceryl monoesters of C₁₆-C₂₂ saturated fatty acids such as glyceryl monostearate, glyceryl monopalmitate, and glyceryl monobehenate. Again, like the sorbitan esters, glyceryl monoester mixtures will typically contain some di- and triester. However, such mixtures should contain predominantly the glyceryl monoester species to be useful in the present invention.

Another class of suitable polyhydroxy fatty acid esters for use in the present invention comprise certain sucrose fatty acid esters, preferably the C₁₂-C₂₂ saturated fatty acid esters of sucrose. Sucrose monoesters and diesters are particularly preferred and include sucrose mono- and di-stearate and sucrose mono- and di- laurate.

Suitable polyhydroxy fatty acid amides for use in the present invention will have the formula: 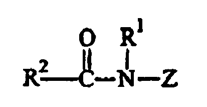 wherein R¹ is H, C₁-C₄ hydrocarbyl, 2-hydroxyethyl, 2-hydroxypropyl, methoxyethyl, methoxypropyl or a mixture thereof, preferably C₁-C₄ alkyl, methoxyethyl or methoxypropyl, more preferably C₁ or C₂ alkyl or methoxypropyl , most preferably C₁ alkyl (i.e., methyl) or methoxypropyl; and R² is a C₅-C₃₁ hydrocarbyl group, preferably straight chain C₇-C₁₉ alkyl or alkenyl, more preferably straight chain C₉-C₁₇ alkyl or alkenyl, most preferably straight chain C₁₁-C₁₇ alkyl or alkenyl, or mixture thereof; and Z is a polyhydroxyhydrocarbyl moiety having a linear hydrocarbyl chain with at least 3 hydroxyls directly connected to the chain. See U.S. patent 5,174, 927 (Honsa), issued December 29, 1992 which discloses these polyhydroxy fatty acid amides, as well as their preparation.

The Z moiety preferably will be derived from a reducing sugar in a reductive amination reaction; most preferably glycityl. Suitable reducing sugars include glucose, fructose, maltose, lactose, galactose, mannose, and xylose. High dextrose corn syrup, high fructose corn syrup, and high maltose corn syrup can be utilized, as well as the individual sugars listed above. These corn syrups can yield mixtures of sugar components for the Z moiety.

The Z moiety preferably will be selected from the group consisting of -CH₂-(CHOH)ₙ-CH₂OH, -CH(CH₂OH)-[(CHOH)ₙ₋₁]-CH₂OH, -CH₂OH-CH₂-(CHOH)₂(CHOR³)(CHOH)-CH₂OH, where n is an integer from 3 to 5, and R³ is H or a cyclic or aliphatic monosaccharide. Most preferred are the glycityls where n is 4, particularly -CH₂-(CHOH)₄-CH₂OH.

In the above formula, R¹ can be, for example, N-methyl, N-ethyl, N-propyl, N-isopropyl, N-butyl, N-2-hydroxyethyl, N-methoxypropyl or N-2-hydroxypropyl. R² can be selected to provide, for example, cocamides, stearamides, oleamides, lauramides, myristamides, capricamides, palmitamides, tallowamides, etc. The Z moiety can be 1-deoxyglucityl, 2-deoxyfructityl, 1-deoxymaltityl, 1-deoxylactityl, 1-deoxygalactityl, 1-deoxymannityl, 1-deoxymaltotriotityl, etc.

The most preferred polyhydroxy fatty acid amides have the general formula: 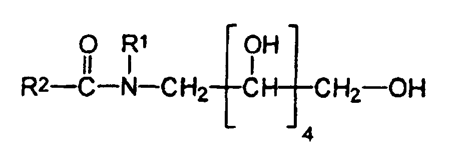 wherein R¹ is methyl or methoxypropyl; R² is a C₁₁-C₁₇ straight-chain alkyl or alkenyl group. These include N-lauryl-N-methyl glucamide, N-lauryl-N-methoxypropyl glucamide, N-cocoyl-N-methyl glucamide, N-cocoyl-N-methoxypropyl glucamide, N-palmityl-N-methoxypropyl glucamide, N-tallowyl-N-methyl glucamide, or N-tallowyl-N-methoxypropyl glucamide.

As previously noted, some of the immobilizing agents may require an emulsifier for solubilization in the emollient. This is particularly the case for certain of the glucamides such as the N-alkyl-N-methoxypropyl glucamides having HLB values of at least about 7. Suitable emulsifiers will typically include those having HLB values below about 7. In this regard, the sorbitan esters previously described, such as the sorbitan stearates, having HLB values of about 4.9 or less have been found useful in solubilizing these glucamide immobilizing agents in petrolatum. Other suitable emulsifiers include steareth-2 (polyethylene glycol ethers of stearyl alcohol that conform to the formula CH₃(CH₂)₁₇(OCH₂CH₂)ₙOH, where n has an average value of 2), sorbitan tristearate, isosorbide laurate, and glyceryl monostearate. The emulsifier can be included in an amount sufficient to solubilize the immobilizing agent in the emollient such that a substantially homogeneous mixture is obtained. For example, an approximately 1:1 mixture of N-cocoyl-N-methyl glucamide and petrolatum that will normally not melt into a single phase mixture, will melt into a single phase mixture upon the addition of 20% of a 1:1 mixture of Steareth-2 and sorbitan tristearate as the emulsifier.

Other types of ingredients that can be used as immobilizing agents, either alone, or in combination with the above-mentioned immobilizing agents, include waxes such as carnauba, beeswax, candelilla, paraffin, ceresin, esparto, ouricuri, rezowax, and other known waxes. Preferably the wax is a paraffin wax. An example of a particularly preferred paraffin wax is Parrafin S.P. 434 from Strahl and Pitsch Inc. P.O. Box 1098 West Babylon, NY 11704.

The amount of the optional immobilizing agent that can be included in the composition will depend on a variety of factors, including the actives (e.g., emollients) involved, the particular immobilizing agent involved, the other components in the composition, whether an emulsifier is required to solubilize the immobilizing agent in the other components, and like factors. When present, the composition will typically comprise from about 5 to about 90% of the immobilizing agent. Preferably, the composition will comprise from about 5 to about 50%, most preferably from about 10 to about 40%, of the immobilizing agent.

Of course, it is highly desirable that at least a portion of the article's structured carrier be made of a hydrophilic material to promote rapid transfer of liquids (e.g., urine) through the structured carrier. Similarly, it may be desirable that the composition be sufficiently wettable to ensure that liquids will transfer through the structured carrier rapidly. Alternatively, hydrophobic skin care composition may be utilized, so long as they are applied such that the fluid handling properties of the structured carrier are adequately maintained. (For example, as discussed below, nonuniform application of the composition to the structured carrier is one means to accomplish this goal.) This diminishes the likelihood that body exudates will flow off the composition-treated structured carrier rather than being drawn through the structured carrier and being absorbed by the absorbent core.

Where a hydrophilic composition is desired, depending upon the particular components used in the composition, a hydrophilic surfactant (or a mixture of hydrophilic surfactants) may, or may not, be required to improve wettability. For example, some immobilizing agents, such as N-cocoyl-N-methoxypropyl glucamide have HLB values of at least about 7 and are sufficiently wettable without the addition of hydrophilic surfactant. Other immobilizing agents such as the C₁₆ - C₁₈ fatty alcohols having HLB values below about 7 may require addition of hydrophilic surfactant to improve wettability when the composition is applied to article structured carriers. Similarly, a hydrophobic emollient such as petrolatum may require the addition of a hydrophilic surfactant if hydrophilic composition is desired. Of course, the concern around wettability is not a factor when the wearer-contacting surface under consideration is other than the article's structured carrier or when fluid handling properties of the structured carrier are adequately maintained via other means (e.g., nonuniform application).

Suitable hydrophilic surfactants will preferably be miscible with the other components of the skin care composition so as to form blended mixtures. Because of possible skin sensitivity of those using disposable absorbent products to which the composition is applied, these surfactants should also be relatively mild and non-irritating to the skin. Typically, these hydrophilic surfactants are nonionic to be not only non-irritating to the skin, but also to avoid other undesirable effects on any other structures within the treated article. For example, reductions in tissue laminate tensile strength, adhesive bond sufficiencies, and the like.

Suitable nonionic surfactants may be substantially nonmigratory after the composition is applied to the article and will typically have HLB values in the range of from about 4 to about 20, preferably from about 7 to about 20. To be nonmigratory, these nonionic surfactants will typically have melt temperatures greater than the temperatures commonly encountered during storage, shipping, merchandising, and use of disposable absorbent products, e.g., at least about 30°C. In this regard, these nonionic surfactants will preferably have melting points similar to those of the immobilizing agents previously described.

Suitable nonionic surfactants for use in compositions that will be applied to the articles, at least in the liquid discharge region of the diaper, include alkylglycosides; alkylglycoside ethers as described in U.S. patent 4,011,389 (Langdon, et al), issued March 8, 1977, alkylpolyethoxylated esters such as Pegosperse 1000MS (available from Lonza, Inc., Fair Lawn, New Jersey), ethoxylated sorbitan mono-, di- and/or tri-esters of C₁₂-C₁₈ fatty acids having an average degree of ethoxylation of from about 2 to about 20, preferably from about 2 to about 10, such as TWEEN 60 (sorbitan esters of stearic acid having an average degree of ethoxylation of about 20) and TWEEN 61 (sorbitan esters of stearic acid having an average degree of ethoxylation of about 4), and the condensation products of aliphatic alcohols with from about 1 to about 54 moles of ethylene oxide. The alkyl chain of the aliphatic alcohol is typically in a straight chain (linear) configuration and contains from about 8 to about 22 carbon atoms. Particularly preferred are the condensation products of alcohols having an alkyl group containing from about 11 to about 22 carbon atoms with from about 2 to about 30 moles of ethylene oxide per mole of alcohol. Examples of such ethoxylated alcohols include the condensation products of myristyl alcohol with 7 moles of ethylene oxide per mole of alcohol, the condensation products of coconut alcohol (a mixture of fatty alcohols having alkyl chains varying in length from 10 to 14 carbon atoms) with about 6 moles of ethylene oxide. A number of suitable ethoxylated alcohols are commercially available, including TERGITOL 15-S-9 (the condensation product of C₁₁-C₁₅ linear alcohols with 9 moles of ethylene oxide), marketed by Union Carbide Corporation; KYRO EOB (condensation product of C₁₃-C₁₅ linear alcohols with 9 moles of ethylene oxide), marketed by The Procter & Gamble Co., the NEODOL brand name surfactants marketed by Shell Chemical Co., in particular NEODOL 25-12 (condensation product of C₁₂-C₁₅ linear alcohols with 12 moles of ethylene oxide) and NEODOL 23-6.5T (condensation product of C₁₂-C₁₃ linear alcohols with 6.5 moles of ethylene oxide that has been distilled (topped) to remove certain impurities), and especially the PLURAFAC brand name surfactants marketed by BASF Corp., in particular PLURAFAC A-38 (a condensation product of a C₁₈ straight chain alcohol with 27 moles of ethylene oxide). (Certain of the hydrophilic surfactants, in particular ethoxylated alcohols such as NEODOL 25-12, can also function as alkyl ethoxylate emollients). Other examples of preferred ethoxylated alcohol surfactants include ICI's class of Brij surfactants and mixtures thereof, with Brij 72 (i.e., Steareth-2) and Brij 76 (i.e., Steareth-10) being especially preferred. Also, mixtures of cetyl alcohol and stearyl alcohol ethoxylated to an average degree of ethoxylation of from about 10 to about 20 may also be used as the hydrophilic surfactant.

Another type of suitable surfactant for use in the composition includes Aerosol OT, a dioctyl ester of sodium sulfosuccinic acid marketed by American Cyanamid Company.

Still another type of suitable surfactant for use in the composition includes silicone copolymers such as General Electric SF 1188 (a copolymer of a polydimethylsiloxane and a polyoxyalkylene ether) and General Electric SF 1228 (a silicone polyether copolymer). These silicone surfactants can be used in combination with the other types of hydrophilic surfactants discussed above, such as the ethoxylated alcohols. These silicone surfactants have been found to be effective at concentrations as low as 0.1%, more preferably from about 0.25 to about 1.0%, by weight of the composition.

The amount of hydrophilic surfactant required to increase the wettability of the composition to a desired level will depend in-part upon the HLB value and level of immobilizing agent, if any, used, the HLB value of the surfactant used and like factors. The composition can comprise from about 0.1 to about 50% of the hydrophilic surfactant when needed to increase the wettability properties of the composition. Preferably, the composition comprises from about 1 to about 25%, most preferably from about 10 to about 20%, of the hydrophilic surfactant when needed to increase wettability.

Compositions can comprise other components typically present in emulsions, creams, ointment, lotions, powders, suspensions, etc. of this type. These components include water, viscosity modifiers, perfumes, disinfectant antibacterial actives, antiviral agents, vitamins, pharmaceutical actives, film formers, deodorants, opacifiers, astringents, solvents, preservatives, and the like. In addition, stabilizers can be added to enhance the shelf life of the composition such as cellulose derivatives, proteins and lecithin. All of these materials are well known in the art as additives for such formulations and can be employed in appropriate amounts in the compositions for use herein.

If water-based skin care compositions are used, a preservative will be needed. Suitable preservatives include propyl paraben, methyl paraben, benzyl alcohol, benzylkonnium, tribasic calcium phosphate, BHT, or acids such as citric, tartaric, maleic, lactic, malic, benzoic, salicylic, and the like. Suitable viscosity increasing agents include some of the agents described as effective immobilizing agents. Other suitable viscosity increasing agents include alkyl galactomannan, silica, talc, magnesium silicate, sorbitol, colloidal silicone dioxide, magnesium aluminum silicate, zinc stearate, wool wax alcohol, sorbiton, sesquioleate, cetyl hydroxy ethyl cellulose and other modified celluloses. Suitable solvents include propylene glycol, glycerine, cyclomethicone, polyethylene glycols, hexalene glycol, diol and multi-hydroxy based solvents. Suitable vitamins include A, D3, E, B5 and E acetate.

In preparing products according to the present invention, the lotion composition is applied to the outer surface (i.e., body facing surface) of an article structured carrier. Any of a variety of application methods that distribute lubricious materials having a molten or liquid consistency can be used. Suitable methods include spraying, printing (e.g., flexographic printing), coating (e.g., gravure coating), extrusion, or combinations of these application techniques, e.g. spraying the composition on a rotating surface, such as a calender roll, that then transfers the composition to the outer surface of the article structured carrier.

Because the structured carrier has an effective open area and a plurality of apertures having a sufficient effective size, urine and low-viscosity fecal material can penetrate the structured carrier regardless of the amount of skin care composition which has been applied to the structured carrier. Accordingly, even if the structured carrier is completely saturated with skin care composition urine and low-viscosity fecal material will readily penetrate therethrough because the very structure of the carrier creates sufficient separation between regions of the applied skin care composition, allowing fluids to penetrate the carrier unobstructed. The problem of occlusion has been solved by providing a structured carrier having an effective open area and a plurality of apertures having a sufficient effective size.

An effective amount of composition needs to be applied to the structured carrier for reducing the adherence of BM to the skin and/or providing a skin benefit to the wearer. The composition is preferably applied to the article structured carrier in an amount ranging from about 0.1 mg/in² to about 35 mg/in². Such levels of composition are believed to be adequate to impart the desired therapeutic and/or protective benefits to the structured carrier.

Since the problem of occlusion has been solved, the composition may be applied to the outer surface of the structured carrier in any manner desired. For example, the composition may be applied to the entire outer surface or only portions thereof. The composition can also be applied nonuniformly to the outer surface of the structured carrier. By "nonuniform" it is meant that the amount, location, pattern of distribution, etc. of the composition can vary over the structured carrier surface. For example, some portions of the treated surface of the structured carrier can have greater or lesser amounts of composition, including portions of the surface that do not have any composition on it.

The composition can be applied to the structured carrier at any point during assembly. For example, the composition can be applied to the structured carrier of the finished disposable absorbent product before it has been packaged. The composition can also be applied to the structured carrier before it is combined with the other raw materials to form a finished disposable absorbent product.

The composition is typically applied from a melt thereof to the article structured carrier. Since the composition melts at significantly above ambient temperatures, it is usually applied as a heated coating to the structured carrier. Typically, the composition is heated to a temperature in the range from about 35_ to about 100_C, preferably from 40_ to about 90_C, prior to being applied to the article structured carrier. Once the melted composition has been applied to the article structured carrier, it is allowed to cool and solidify to form solidified coating or film on the surface of the structured carrier. Preferably, the application process is designed to aid in the cooling/set up of the composition.

The diaper 20 may further comprise elasticized leg cuffs (not shown) which provide improved containment of liquids and other body exudates. Each elasticized leg cuff may comprise several different embodiments for reducing the leakage of body exudates in the leg regions. (The leg cuff can be and is sometimes also referred to as leg bands, side flaps, barrier cuffs, or elastic cuffs.) U.S. Patent 3,860,003 describes a disposable diaper 20 which provides a contractible leg opening having a side flap and one or more elastic members to provide an elasticized leg cuff (gasketing cuff). Commonly assigned U.S. Patent 4,909,803 entitled "Disposable Absorbent Article Having Elasticized Flaps" issued to Aziz et al. on March 20, 1990, describes a disposable diaper 20 having "stand-up" elasticized flaps (barrier cuffs) to improve the containment of the leg regions. Commonly assigned U.S. Patent 4,695,278 entitled "Absorbent Article Having Dual Cuffs" issued to Lawson on September 22, 1987, describes a disposable diaper 20 having dual cuffs including a gasketing cuff and a barrier cuff.

The diaper 20 preferably further comprises an elastic waist feature (not shown) that provides improved fit and containment. The elastic waist feature is that portion or zone of the diaper 20 which is intended to elastically expand and contract to dynamically fit the wearer's waist The elastic waist feature at least extends longitudinally outwardly from at least one of the waist edges of the absorbent core 28 and generally forms at least a portion of the end edge of the diaper 20. Disposable diapers are generally constructed so as to have two elastic waist features, one positioned in the first waist region 27 and one positioned in the second waist region 29, although diapers can be constructed with a single elastic waist feature. Further, while the elastic waist feature or any of its constituent elements can comprise a separate element affixed to the diaper 20, the elastic waist feature is preferably constructed as an extension of other elements of the diaper 20 such as the backsheet 26 or the structured carrier 24, preferably both the backsheet 26 and the structured carrier 24. The elasticized waistband 34 may be constructed in a number of different configurations including those described in U.S. Patent 4,515,595 issued to Kievit et al. on May 7, 1985.

The diaper 20 also comprises a fastening system 36 which forms a side closure which maintains the first waist region 27 and the second waist region 29 in an overlapping configuration such that lateral tensions are maintained around the circumference of the diaper 20 to maintain the diaper 20 on the wearer. Exemplary fastening systems are disclosed in U.S. Patent 4,846,815 entitled "Disposable Diaper Having An Improved Fastening Device" issued to Scripps on July 11, 1989; U.S. Patent 4,894,060 entitled "Disposable Diaper With Improved Hook Fastener Portion" issued to Nestegard on January 16, 1990; commonly assigned U.S. Patent 4,946,527 entitled "Pressure-Sensitive Adhesive Fastener And Method of Making Same" issued to Battrell on August 7, 1990; commonly assigned U.S. Patent 3,848,594 entitled "Tape Fastening System for Disposable Diaper" issued to Buell on November 19, 1974; commonly assigned U.S. Patent B1 4,662,875 entitled "Absorbent Article" issued to Hirotsu et al. on May 5, 1987.

The diaper 20 is preferably applied to a wearer by positioning one of the waist regions, preferably the second waist region 29, under the wearer's back and drawing the remainder of the diaper 20 between the wearer's legs so that the other waist region, preferably the first waist region 27, is positioned across the front of the wearer. The tape tabs 36 of the fastening system are then released from the release portion. The diaperer then wraps the elasticized side panel around the wearer, while still grasping the tab portion. The fastening system is secured to the outer surface of the diaper 20 to effect two side closure.

## Claims

1. A disposable absorbent article (20) comprising:
a liquid pervious structured carrier (24) comprising a non-woven web having an inner surface oriented toward the interior of said disposable absorbent article (20) and an outer surface oriented toward the skin of the wearer when the disposable absorbent article (20) is worn, said structured carrier (24) having an effective open area of at least about 12 percent and a plurality of apertures with an effective size greater than 2.0 square millimeters, said outer surface of said structured carrier (24) comprising an effective amount of a skin care composition which is semi-solid or solid at 20 °C and which is partially transferable to the wearer's skin;
a liquid impervious backsheet (26) at least partially peripherally joined to said structured carrier (24); and
an absorbent core (28) intermediate said structured carrier (24) and said backsheet (26).

2. The disposable absorbent (20) article according to Claim 1 wherein said structured carrier (24) has an effective open area of at least about 15 percent.

3. The disposable absorbent article (20) according to Claim 1 wherein said structured carrier (24) has an effective open area of at least about 20 percent.

4. The disposable absorbent article (20) according to Claim 1 wherein said structured carrier (24) has an effective open area of at least about 30 percent.

5. The disposable absorbent article (20) of Claim 1 wherein the skin care composition comprises a petroleum based emollient selected from the group consisting of mineral oil, petrolatum, and mixtures thereof.

6. The disposable absorbent article (20) of Claim 1 wherein said skin care composition comprises an immobilizing agent, said immobilizing agent having a melting point of at least about 40°C.

7. The disposable absorbent article (20) of Claim 6 wherein said immobilizing agent is selected from the group consisting of polyhydroxy fatty esters, polyhydroxy fatty acid amides, C₁₄-C₂₂ fatty alcohols, C₁₂-C₂₂ fatty acids, C₁₂-C₂₂ fatty alcohol ethoxylates, and mixtures thereof.

8. The disposable absorbent article (20) of Claim 7 wherein said immobilizing agent comprises a C₁₆-C₁₈ fatty alcohol selected from the group consisting of cetyl alcohol, stearyl alcohol, and mixtures thereof.

9. The disposable absorbent article (20) of Claim 5 wherein said immobilizing agent comprises a polyhydroxy fatty acid ester having the formula: 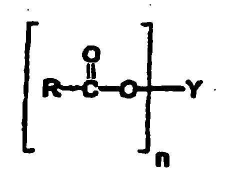 wherein R is a C₅-C₃₁ hydrocarbyl group; Y is a polyhydroxyhydrocarbyl moiety having a hydrocarbyl chain with at least 2 free hydroxyls directly connected to the chain; and n is at least 1.

10. The disposable absorbent article (20) of Claim 5 wherein said immobilizing agent comprises a polyhydroxy fatty acid amide having the formula: 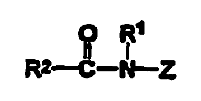 wherein R¹ is H, C₁-C₄ hydrocarbyl, 2-hydioxyethyl, 2-hydroxypropyl, methoxyethyl, methoxypropyl or a mixture thereof; R² is a C₅-C₃₁ hydrocarbyl group; and Z is a polyhydroxyhydrocarbyl moiety having a linear hydrocarbyl chain with at least 3 hydroxyls directly connected to the chain.

11. The disposable absorbent article (20) of Claim 5 wherein said immobilizing agent comprises a paraffin wax.

12. The disposable absorbent article (20) of Claim 1 wherein said skin care composition comprises a polysiloxane emollient having a plastic or fluid consistency at 20°C.

13. The disposable absorbent article (20) of Claim 1 wherein said skin care composition comprises a polyol polyester emollient.

## Patentansprüche

1. Absorbierender Einwegartikel (20) mit:
einem flüssigkeitsdurchlässigen, strukturierten Träger (24) mit einer Nonwoven-Bahn, die eine zur Innenseite des absorbierenden Einwegartikels (20) orientierte Innenfläche und eine zur Haut des Trägers orientierte Außenfläche aufweist, wenn der absorbierende Einwegartikel (20) getragen wird, wobei der strukturierte Träger (24) eine effektive offene Fläche von wenigstens etwa 12 Prozent und eine Mehrzahl von Öffnungen mit einer effektiven Größe größer als 2,0 Quadratmillimeter hat, wobei die Außenfläche des strukturierten Trägers (24) eine effektive Menge einer Hautpflegezusammensetzung aufweist, welche bei 20°C halbfest oder fest ist und welche auf die Haut des Trägers partiell übertragbar ist;
einer flüssigkeitsundurchlässigen Außenlage (26), die wenigstens teilweise umfänglich mit dem strukturierten Träger (24) verbunden ist; und
einem absorbierenden Kern (28) zwischen dem strukturierten Träger (24) und der Außenlage (26).

2. Absorbierender Einwegartikel (20) nach Anspruch 1, in welchem der strukturierte Träger (24) eine effektive offene Fläche von wenigstens etwa 15 Prozent hat.

3. Absorbierender Einwegartikel (20) nach Anspruch 1, in welchem der strukturierte Träger (24) eine effektive offene Fläche von wenigstens etwa 20 Prozent hat.

4. Absorbierender Einwegartikel (20) nach Anspruch 1, in welchem der strukturierte Träger (24) eine effektive offene Fläche von wenigstens etwa 30 Prozent hat.

5. Absorbierender Einwegartikel (20) nach Anspruch 1, in welchem die Hautpflegezusammensetzung ein auf Erdöl basierendes Emolliens ausgewählt aus der Gruppe bestehend aus Mineralöl, Vaseline und Mischungen daraus umfasst.

6. Absorbierender Einwegartikel (20) nach Anspruch 1, in welchem die Hautpflegezusammensetzung ein Immobilisierungsmittel umfasst, wobei das Immobilisierungsmittel einen Schmelzpunkt von wenigstens etwa 40°C hat.

7. Absorbierender Einwegartikel (20) nach Anspruch 6, in welchem das Immobilisierungsmittel ausgewählt ist aus der Gruppe bestehend aus Polyhydroxy-Fettester, Polyhydroxy-Fettsäureamide, C₁₄-C₂₂ Fettalkohole, C₁₂-C₂₂ Fettsäuren, C₁₂-C₂₂ Fettalkoholethoxylate und Mischungen daraus.

8. Absorbierender Einwegartikel (20) nach Anspruch 7, in welchem das Immobilisierungsmittel einen C₁₆-C₁₈ Fettalkohol ausgewählt aus der Gruppe bestehend aus Cetylalkohol, Stearylalkohol und Mischungen daraus umfasst.

9. Absorbierender Einwegartikel (20) nach Anspruch 5, in welchem das Immobilisierungsmittel einen Polyhydroxy-Fettsäureester mit der Formel umfasst: 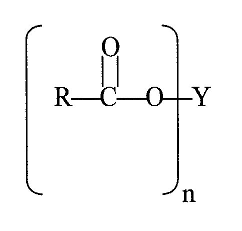 in welcher R eine C₅-C₃₁ Hydrocarbyl-Gruppe ist; Y ein Polyhydroxyhydrocarbyl-Anteil mit einer Hydrocarbyl-Kette mit wenigstens zwei freien Hydroxylen ist, die direkt mit der Kette verbunden sind; und n wenigstens 1 ist.

10. Absorbierender Einwegartikel (20) nach Anspruch 5, in welchem das Immobilisierungsmittel ein Polyhydroxy-Fettsäureamid mit der Formel umfasst: 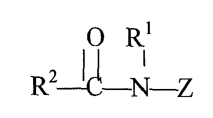 in welcher R¹ H ist, C₁-C₄ Hydrocarbyl, 2-hydroxyethyl. 2-hydroxypropyl, Methoxyethyl, Methoxypropyl oder ein Gemisch daraus ist; R² eine C₅-C₃₁ Hydrocarbyl-Gruppe ist; und Z ein Polyhydroxyhydrocarbyl-Anteil mit einer linearen Hydrocarbyl-Kette mit wenigstens 3 Hydroxylen ist, die direkt mit der Kette verbunden sind.

11. Absorbierender Einwegartikel (20) nach Anspruch 5, in welchem das Immobilisierungsmittel ein Paraffinwachs umfasst.

12. Absorbierender Einwegartikel (20) nach Anspruch 1, in welchem die Hautpflegezusammensetzung ein Polysiloxan-Emolliens mit einer plastischen oder fluiden Konsistenz bei 20°C umfasst.

13. Absorbierender Einwegartikel (20) nach Anspruch 1, in welchem die Hautpflegezusammensetzung ein Polyolpolyester-Emolliens umfasst.

## Revendications

1. Article absorbant jetable (20) comprenant :
un support structuré, perméable aux liquides (24), comprenant un voile de non-tissé ayant une surface interne orientée vers l'intérieur dudit article absorbant jetable (20) et une surface externe orientée vers la peau de l'utilisateur lorsque l'article absorbant jetable (20) est porté, ledit support structuré (24) ayant une surface de vide efficace d'au moins environ 12 % et une pluralité d'ouvertures de dimension effective supérieure à 2 mm², ladite surface externe dudit support structuré (24) comprenant une quantité efficace d'une composition de soin de la peau qui est solide ou semi-solide à 20°C et qui est partiellement transférable sur la peau de l'utilisateur,
une feuille de fond imperméable aux liquides (26) au moins partiellement soudée en périphérie audit support structuré (24), et
un noyau absorbant (28) intercalé entre ledit support structuré (24) et ladite feuille de fond (26).

2. Article absorbant jetable (20) selon la revendication 1, dans lequel ledit support structuré (24) présente une surface de vide efficace d'au moins environ 15 %.

3. Article absorbant jetable (20) selon la revendication 1, dans lequel ledit support structuré (24) présente une surface de vide efficace d'au moins environ 20%.

4. Article absorbant jetable (20) selon la revendication 1, dans lequel ledit support structuré (24) présente une surface de vide efficace d'au moins environ 30%.

5. Article absorbant jetable (20) selon la revendication 1, dans lequel la composition de soin de la peau comprend un émollient à base de vaseline choisi dans le groupe comprenant l'huile minérale, la vaseline, et leurs mélanges.

6. Article absorbant jetable (20) selon la revendication 1, dans lequel ladite composition de soin de la peau comprend un agent d'immobilisation, ledit agent d'immobilisation ayant un point de fusion d'au moins environ 40°C.

7. Article absorbant jetable (20) selon la revendication 6, dans lequel ledit agent d'immobilisation est choisi dans le groupe comprenant les esters gras polyhydroxylés, les amides d'acides gras polyhydroxylés, les alcools gras en C₁₄-C₂₂, les acides gras en C₁₂-C₂₂, les produits d'éthoxylation d'alcools gras en C₁₂-C₂₂, et leurs mélanges.

8. Article absorbant jetable (20) selon la revendication 7, dans lequel ledit agent d'immobilisation comprend un alcool gras en C₁₆-C₁₈ choisi dans le groupe comprenant l'alcool cétylique, l'alcool stéarylique, et leurs mélanges.

9. Article absorbant jetable (20) selon la revendication 5, dans lequel ledit agent d'immobilisation comprend un ester d'acide gras polyhydroxylé répondant à la formule : 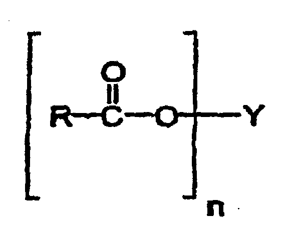 dans laquelle R est un groupe hydrocarbyle en C₅-C₃₁, Y est un fragment polyhydroxyhydrocarbyle ayant une chaîne hydrocarbyle avec au moins deux hydroxyles libres reliés directement à la chaîne et n vaut au moins 1.

10. Article absorbant jetable (20) selon la revendication 5, dans lequel ledit agent d'immobilisation comprend un amide d'acide gras polyhydroxylé répondant à la formule : 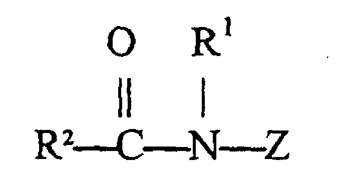 dans laquelle R¹ représente l'hydrogène, un radical hydrocarbyle en C₁-C₄, 2-hydroxyéthyle, 2-hydroxypropyle, méthoxyéthyle, méthoxypropyle ou un mélange de ceux-ci, R est un radical hydrocarbyle en C₅-C₃₁ et Z est un fragment polyhydroxyhydrocarbyle ayant une chaîne hydrocarbyle linéaire avec au moins trois hydroxyles reliés directement à la chaîne.

11. Article absorbant jetable (20) selon la revendication 5, dans lequel ledit agent d'immobilisation comprend une cire de paraffine.

12. Article absorbant jetable (20) selon la revendication 1, dans lequel ladite composition de soin de la peau comprend un émollient de polysiloxane ayant une consistance plastique ou fluide à 20°C.

13. Article absorbant jetable (20) selon la revendication 1, dans lequel ladite composition de soin de la peau comprend un émollient de polyester-polyol.
